# EUROPEAN PATENT APPLICATION

(11) **EP 4 686 504 A1**
(43) Date of publication of application: **04.02.2026**
(21) Application number: 24192470.3
(22) Date of filing: 01.08.2024
(51) Int. Cl.: B01J 2/22, B29C 33/00, B29C 35/16, A61K 9/19, A61K 9/20, F26B 5/06

(54) **METHOD FOR THE MANUFACTURE OF FROZEN PELLETS AND A PRODUCT IN THE FORM OF FROZEN PELLETS**

(71) Applicant: Jena Biotech Invest GmbH, 07745 Jena (DE)
(72) Inventor: HELLMICH-DUONG, Thanh Tu, 07745 Jena (DE); HERMANN, Hanno, 07751 Jena (DE)
(74) Representative: Schiweck Weinzierl Koch Patentanwälte Partnerschaft mbB

(57) **Abstract**

In various embodiments a method for the manufacture of frozen pellets is provided, comprising: (a) applying a predefined quantity of a liquid composition to a respective receiving surface of a plurality of receiving structures, (b) freezing the liquid composition, thereby generating a plurality of frozen pellets, wherein the receiving surface of the receiving structure has a concave shape; and wherein said liquid composition comprises one or more saccharide. In various further embodiments a product is provided, in the form of a plurality of frozen pellets, obtainable by the method of any one of the preceding claims.

## Description

The present patent application relates to a method for the manufacture of frozen pellets and, in particular, to the manufacture of freeze-dried pellets. Furthermore, the present patent application relates to a product, in the form of a plurality of frozen pellets, obtainable by the method.

Biological and biochemical materials of various kinds, in particular bioactive substances, for use in the field of life sciences often come in the form of lyophilised beads. Those small, freeze-dried particles, between a few hundreds of micrometers and a few millimeters in size, which are also commonly referred to as lyo beads (lyo pellets) or dried cryo beads (cryo pellets), offer numerous advantages, such as stable storage of the active ingredients at room temperature. The dried cryo beads allow for transportability of originally sensitive materials without the mandatory adherence to cooling chains and thus enable their usability in processes and/or laboratories in countries with incomplete or missing cooling infrastructure.

In order to achieve the most favourable ratio of mechanical stability and volume to the surface of such cryo beads, efforts are undertaken to manufacture them in round forms. In this form, the lyo-beads are most stable against mechanical stress and due to their compact shape, can be transported and used easily, without loss of material due to abrasion and in a space-saving manner.

From the prior art, three main methods for the manufacture of cryo beads are known. Following an initial methodology, ice particles, e.g. dry ice pellets, are pressed into balls or pellets by means of punches or punch pairs and matrices. However, this method is technologically difficult to master for use in the pharmaceutical and diagnostic environment.

A second manufacturing method involves freezing of a reagent mixture in or on liquid nitrogen or other cryogenic liquids, such as silicone oils. If the volumes of the reagent mixture are sufficiently small, the liquid droplets initially float on the evaporating nitrogen as a result of the "inverse Leidenfrost effect" and then solidify. By cooling the liquid droplets, the temperature difference to the still boiling nitrogen decreases, so that the droplet sinks. If a liquid droplet is too large or too heavy, the evaporating nitrogen is not capable of holding the droplet on the surface. As it sinks down, the liquid then solidifies in the form of droplets. This is especially true for non-boiling cryogenic liquids, such as silicone oil.

As a result of the boiling of the nitrogen, small volumes, so-called "satellite droplets", can be detached from a still liquid droplet, which leads to a both undesirable and uncontrollable decrease in the volume of the droplet. Furthermore, because of the boiling nitrogen, the droplets floating on the nitrogen vapor move around and merge into undesirably large droplets when colliding with other droplets. This results in a mixture of unevenly sized beads, i.e. an inhomogeneous size distribution of the beads. In addition, before being completely frozen, the drops come into direct contact with nitrogen and possibly impure substances or agents contained therein which may contaminate the product. Last but not least, during the manufacture of a batch of cryo beads by means of a liquid nitrogen bath, depending on their time of their formation the cryo beads are exposed to the temperature of -196°C for different amounts of time, which may lead to an inhomogeneous distribution of the internal structure of the cryo beads throughout the batch.

In a third approach, drops of the solution to be processed into a cryo bead are applied to an (ultra)cold flat plate, on which they freeze. To support a formation of round or sphere-like forms the plate can have repulsive, especially hydrophobic, properties at least in the relevant areas of its surface. This method provides cryo beads which are hemispheres, spherical segments, or flattened, elliptical lenses - spherical shapes or spheres cannot be obtained due to the gravitational force acting on the volumes resting on the (ultra)cold plate.

Examples of the production and optional drying of the cryo beads can be found in documents US 4,848,094, US 2014/0294872 A1, US 2016/0252300 A1, WO 2009/092703 A1, WO 2010/125087 A1 and WO 2013/066769 A1.

The objective technical problem to be solved by the subject-matter of the present patent application may be seen in the provision of an improved way of producing frozen pellets and, accordingly, in the provision of frozen pellets.

In various embodiments of the present invention a method for the manufacture of frozen pellets is provided. The method includes (a) applying a predefined quantity of a liquid composition to a respective receiving surface of a plurality of receiving structures, and (b) freezing the liquid composition, thereby generating a plurality of frozen pellets, wherein the receiving surface of the receiving structure has a concave shape, and wherein said liquid composition comprises one or more saccharide.

In the context of this description the term frozen pellet can be understood in particular as a frozen or cryogenic pellet/bead, which comprises the frozen liquid composition. The liquid composition may comprise various active substances, in particular therapeutic agents, excipients, enzymes, proteins, genetic material, in particular sections of DNA and/or RNA, primers or oligonucleotides, salts, organic substances, complexes and/or other substances or molecules, vesicles, chromosomes, cell organelles and complete cells, in the present case also as reagent mixture.

The device for use in the method according to various embodiments may comprise a carrier, such as a plate, comprising a plurality of receiving structures. In that sense, the carrier may be structured with a plurality of receiving structures. Each receiving structure comprises the concave receiving surface which may form an upper surface of the receiving structure. Each receiving surface defines a receiving volume with respect to the liquid composition, wherein the receiving volume is configured to receive and hold a certain larger volume of the liquid composition and prevent undesired flowing or rolling away.

In certain cases, the receiving surfaces may optionally have a coating and/or surface structure that is slightly to moderately repellent to the liquid composition, i.e. in particular hydrophobic, at least on the receiving surface, in order to further support the formation of a preferably spherical volume of the liquid composition and to facilitate removal of the pellets once they are frozen. The concave form of the receiving surface also helps to position the still liquid sample and represents a mechanical spatial resistance against the liquid composition sample rolling away.

The receiving structures may comprise posts (with a concave top surface) or other block-like structures which extend upwards from a surface of the carrier and are arranged in a pattern on the surface thereof. In such a case, the surface of the carrier is uneven. Alternatively, the receiving structures may be provided in recesses of the surface of the carrier, each recess having a surface area which is larger than a surface area of the receiving structure. In a side view, the receiving structures may extend above the surface of the carrier, or they may be accommodated in the recesses such that in a side view they do not extend above the surface of the carrier. In any case, the receiving structures may be formed such that in a top view their perimeter coincides with the perimeter of the receiving surface. Expressed in other words, the receiving structures may have a sharp top perimeter, corresponding to an upper edge of a sidewall of their side wall and simultaneously to the perimeter of the receiving surface. Expressed in yet other words, each of the receiving structures may correspond to an exposed structure in the sense that it protrudes from the surface directly surrounding it.

To achieve freezing of the liquid composition received in the receiving surfaces, the carrier may be tempered, in particular cooled. Thus, the materials of the carrier may be those that allow effective cooling of the receiving structures, such as metals and metallic alloys, composites, especially with metallic layers, i.e. materials with a high heat conduction. The carrier may be cooled by thermal contact with a cold sink, such as liquid nitrogen. By modulating the coupling therebetween the temperature of the carrier may be adjusted. In addition, the carrier may include tempering elements, such as electric wires and/or Peltier elements, to facilitate fast tempering thereof.

The liquid composition may include various aqueous solutions of different viscosities, also including gels, in particular, if due to molecular interactions they are able to form a round volume, preferably spheres under suitable conditions. In particular, the liquid composition may include a biological material and/or inorganic material and/and organic material. The liquid may be applied to the receiving surfaces by means of a pipetting device, such as a pipetting robot. Preferably, the pipetting device may comprise a number of pipettes which corresponds to the number of receiving surfaces being processed simultaneously. The volumes of the liquid composition provided on the receiving surfaces may be in the range of 1-30 µl.

According to further embodiments of the method, in step (b) the plurality of frozen pellets may be generated in parallel. This means that the processing of the plurality of liquid composition volumes may take place in a spatially parallel manner, i.e. distributed over the area carrying the receiving surfaces. At any given moment, the same manufacturing step may take place at all of the multiple receiving surfaces.

According to further embodiments of the method, in step (b) the plurality of frozen pellets may be generated in a synchronized manner. This means that the processing of the plurality of each of the liquid composition volumes may take place at the same time and at the same pace.

According to further embodiments of the method, the receiving surface has a shape corresponding to a spherical cap. The size of the spherical cap may be varied and may be any practical size providing sufficient support for the volume of liquid composition arranged thereon and a hemisphere. In other words, the radius of the base of the spherical cap may range between a value larger than zero p to the radius of the corresponding sphere. The perimeter of the base of the cap spans a circular surface which may define top surface of the receiving structure.

According to further embodiments of the method, the receiving surface in step (b) the receiving surfaces of the plurality of receiving structures may be at a predefined temperature which is in the range of +/-80 K, preferably in the range of +/-50 K, more preferably in the range of +/-30 K, and more preferably in the range of +/-10 K of the freezing temperature, the eutectic temperature or glass transition temperature of the liquid composition. The choice of the freezing temperature, the eutectic temperature or glass transition temperature of the liquid composition as the reference temperature for setting the temperature of the receiving spheres may depend on the liquid composition being used and its concentration. In particular, if the liquid composition comprises a cryoprotectant, the glass transition temperature may be the most relevant temperature to be considered for choosing the temperature of the receiving surfaces.

In contrast to methods relying on freezing of a liquid composition in or on liquid nitrogen or other cryogenic liquids (will be referred to as liquid nitrogen method hereinafter), the method of the present invention allows to set the temperature of the receiving surfaces to a desired value, which may be an optimized value for a given liquid composition. The present manufacturing method provides for a continuous "single-phase freezing" through direct contact of the liquid composition with the surface of the receiving surface which gradually solidifies. In contrast thereto, the liquid nitrogen method corresponds to a "two-phase freezing" method, where the freezing process is discontinuous: First a liquid droplet cools on a steam pad of evaporated liquid nitrogen and the process is defined by a first, relatively flat cooling rate. Subsequently, the pre-cooled liquid droplet comes into direct contact with the liquid nitrogen, the process being defined by a second, relatively steep cooling rate.

According to further embodiments, the method may include increasing or lowering the temperature of the receiving surfaces from the predefined temperature for a predetermined period of time. Expressed differently, the method of the present invention may include exposing the pellets, during freezing (i.e. during step (b)) or once they are frozen, to a different temperature (higher/lower) in a controlled manner, i.e. with a predetermined temperature transition rate (heating/cooling) and for a predetermined period of time. This further process may be used to influence the structure of the frozen pellets in a certain manner.

According to further embodiments of the method, the receiving surfaces may define a volume which corresponds to or is less than 50% of the volumes of the liquid composition provided on the receiving surfaces. In other words, the receiving surface, acting as support for the volume of the liquid composition, may exert a varying amount of "grasp" thereon. The depth of the receiving surface, which may be quantified by the distance between a flat surface covering its opening and a point lying farthest away therefrom (in the case of a spherical cap the depth corresponds to the height thereof), may be adjusted to the volume of the liquid composition and to the nature thereof, in particular its surface tension. Generally, the depth of the receiving surface may be adjusted such that the target volume of the liquid composition arranged thereof does not run away/dissipate.

According to further embodiments of the method step (a) may comprises applying a first liquid composition and at least a second liquid composition, simultaneously or successively, to the receiving surfaces. By doing so, dual-phase pellets may be manufactured, which comprise two different liquid compositions which are substantially separated from one another. For example, by applying a second liquid composition on a first liquid composition, a frozen pellet may be generated which comprises a more or less round core of the first liquid composition embedded in the second liquid composition. The outer form of the dual-phase frozen pellet may correspond to an outer form of a single-phase frozen pellet which was manufactured with a volume of a single liquid composition which is equal to the sum of the volume of the first liquid composition and the second liquid composition.

According to further embodiments, the method may additionally include removing the frozen pellets from the receiving surfaces. For this process step a removal device may be used, which may substantially comprise a removal plate which is moved relative to the plurality of receiving surfaces to exert a force on the frozen pellets, thereby lifting/removing the frozen pellets from their "seats". This step may be performed in preparation of a subsequent lyophilization process.

According to further embodiments, the method may additionally include arranging the removed frozen pellets in a container. After being arranged in the container, the frozen pellets correspond to a bulk material. The frozen pellets may be arranged in the container in layers.

In order to store, transport and use the frozen pellets for extended periods of time without further cooling, they may be subsequently freeze-dried (lyophilised) in further embodiments of the method in order to remove the water contained in the frozen liquid composition. The frozen pellets may be freeze dried on the receiving surfaces or after having been removed and arranged in a container. In this context, the freeze-dried pellets may be referred to as lyo pellets.

According to further embodiments of the method, the shape and/or the volume of the frozen pellets and the shape and/or volume of the dried frozen pellets are substantially the same. Thus, the method of various embodiments may be characterized by the constancy of the shape and/or volume of the pellets over the entire process. That is, the shape and/or the volume of the liquid composition may be substantially the same as the shape and/or volume of the cryo pellet and the latter may be substantially the same as the shape and/or volume of the lyo pellet (dry volume), except for minor shrinkage processes which can be neglected.

Furthermore, in the method, according to various embodiments, the variability of the manufactured beads as the end product - be it cryo beads or lyo beads - substantially corresponds to the variability of the volumes of the liquid composition provided on the receiving surfaces, since inhomogeneities within a manufactured batch of pellets may be practically excluded. Thus, the source of variability of the end product is reduced to the (in)accuracy of the employed pipetting device, which in state of the art pipetting devices lies in the range of 1-2 % for volumes of 5-20 µl.

In the context of this description, a batch of manufactured frozen pellets may refer to pellets which are manufactured at the same time in parallel in a corresponding device. The number of frozen pellets may be limited by the size of the pipetting device and/or the size of the carrier and the number of receiving surfaces provided thereon, in general, such that generally the method of the present invention is characterized by good scalability.

According to further embodiments of the method, the frozen pellets and the dried frozen pellets have a spherical shape. The spherical shape may be achieved by using receiving surfaces which have the shape of a spherical cap and adjusting the volume of the liquid composition provided thereon to the volume of the spherically shaped receiving surface. That is, the volume of the liquid composition provided on the spherical receiving surfaces may substantially correspond to the volume a sphere matching the curvature of the spherical cap formed by the receiving surface.

The method of the present invention allows for the reliable manufacture of a plurality of frozen pellets of spherical shape. The spherical shape provides for a good rollability of the frozen and also freeze-dried pellets, which is a valuable technical quality. Spherical pellets are easy to transport over hose liens, open gutters and inclined planes. The good rollability and the lack of edges minimizes tumbling and loss of material caused abrasion upon contact with other frozen and freeze-dried pellets. Those effects may be particularly exploited in the production of pharmaceutical drugs, since the method of the present invention may be used to manufacture freeze-dried pellets with a low friability (abrasion resistance). The sphericity of the frozen pellets also facilitates processing via vacuum tweezers (pick and place) due to isotropic eligibility to be subjected to the suction of the vacuum tweezers.

According to further embodiments of the method, the frozen and the dried frozen pellets have a sphericity of at least 0.97. Those embodiments require the receiving surfaces to have the form of a spherical cap. Due to the nature of the manufacturing method of the present invention, the sphericity of the manufactured frozen and/or freeze-dried pellets may be substantially the same for all the pellets in the manufactured ensemble. In other words, there is no inter-pellet inhomogeneity of the shape or sphericity (and other parameters/features of the manufactured pellets) at least within one manufactured batch.

According to further embodiments of the method, the frozen pellets may have a first visible mark dividing their surface into two spherical caps, the first visible mark preferably comprising a line. In the case of spherical receiving surfaces the first visible mark may correspond to a great circle. The first visible mark may further correspond to an imprint of the perimeter or rim of the receiving surface on the surface of the frozen pellet. The same may apply to the lyophilised pellet.

Overall, especially depending on the ratio of the volume of the liquid composition provided on the receiving surfaces and the volumes of spheres defining the spherical receiving surfaces, the two spherical caps divided by the first visible value may have slightly different radii. Thus, the first visible mark may be caused by one spherical cap slightly overlapping the other spherical cap. That is, at the level of the visible mark dividing the surface of the frozen and preferably also freeze-dried pellet into two spherical caps those two spherical caps. The curvatures of the spherical caps may be harmonized by choosing the volume of the liquid composition placed on the receiving surface to match the volume of a sphere defining the spherical receiving surface. An overfilling or underfilling may lead to the situation as described above, where the two spherical caps have slightly different radii.

According to further embodiments of the method, the at least one, optionally all receiving surfaces comprise an emboss which is configured to leave a corresponding second visible mark on the surface of the respective frozen pellet, and preferably also the freeze-dried pellet. The emboss is provided to leave an imprint in the form of the second visible mark on the surface of the respective frozen pellet. The emboss may be negative or positive, i.e. it may be embedded in the surface of the at least one receiving surface or it may protrude from the surface of the receiving surface. The providing of the emboss on at least one of the receiving surfaces allows for "stamping" the preferably spherical frozen pellets and consequently also freeze-dried pellets. The second visible mark may be provided on the surface of the at least one frozen pellet without having to execute an additional dedicated manufacturing step to do so, since the embossing of the at least one frozen pellet is seamlessly merged into step (b) of the manufacturing method of the present invention. The second visible mark may include a logo of a company, a charge ID, a warning sign, etc. The emboss may be configured to also exert a holding force on the frozen pellet, which prevents the pellet from easily rolling out of the holding surface, in particular in the case of relatively shallow holding surfaces. For that purpose, the emboss may comprise a rod or a cone protruding externally from the holding surface.

According to further embodiments of the method, the plurality of frozen pellets includes at least 8, preferably at least 12, more preferably at least 16, even more preferably at least 24, even more preferably at least 96, even more preferably at least 384, even more preferably at least 768 frozen pellets or more. In the case of a carrier carrying 768 receiving surfaces, 1 Mio. per day frozen pellets may be manufactured in one batch. Generally, the method of the present invention is configured to manufacture a vast number of frozen pellets in a parallel (spatially) and synchronous (temporally) manner. The indicated numbers are motivated by standard sizes of well-plates according to the SBS (Society for Biomolecular Screening) industry standard, which may be used for during the manufacture according to the present invention. Of course, it should be realized that the indicated number are merely provided to illustrate the potential of the method of the present invention and not to limit it in any sense. Thus, the person skilled in the art will understand that other formats of manufacturing equipment may be used, which may be according to a different standard or not standardized at all.

According to further embodiments of the method, the plurality of frozen and subsequently dried pellets may have a homogenous porosity. The homogenous porosity refers to the distribution of porosity patterns throughout at least one batch, preferably through multiple batches. Expressed differently, the porosity pattern or the internal structure of a frozen pellet of any two pellets from one batch, and preferably from different batches of the manufacturing method of the present invention is the same. This is enabled by the method of the present invention which allows for tight control of the manufacturing conditions and may be repeated in a homogenous manner multiple times. Furthermore, stochastic processes, such as the "dual-phase freezing" generally involved in a liquid nitrogen process and the "inverse Leidenfrost effect" in particular, are substantially avoided. Due to the absence of such effects which may alter the process path during the manufacturing process in an unpredictable manner, the same result with regard to the porosity (and also shape and volume) of the frozen pellets may be obtained repeatedly.

In various further embodiments of the present invention, a product is provided, in the form of a plurality of frozen pellets, obtainable by the method of any one of the embodiments described above. The plurality of frozen pellets may be obtained by the method of any one of the embodiments described above. Therefore, the technical properties of the method described above may be imparted on the product, such that the plurality of frozen and preferably also freeze-dried pellets may include structural and spatial features corresponding to or imparted by features of the method for the manufacture of frozen pellets according to various embodiments.

The plurality of frozen pellets provided pellets may be freeze-dried, as described above with regard to certain embodiments of the method of various embodiments, to provide a plurality of freeze-dried pellets.

According to various embodiments of the product, the plurality of frozen pellets may substantially have the same porosity.

According to various embodiments of the product, the plurality of frozen pellets may include at least 8, preferably at least 12, more preferably at least 16, even more preferably at least 24, even more preferably at least 96, even more preferably at least 384, even more preferably at least 768 frozen pellets or more, all having the same spherical shape and the same porosity.

According to various embodiments of the product, the frozen pellets include frozen and subsequently dried, preferably freeze-dried, pellets.

As noted before, further features of the product may be derived from the various embodiments of the manufacturing process described beforehand.

According to the present invention, said liquid composition in the product according to the present invention and as used in the method according to the present invention comprises one or more saccharide.

Said saccharide may be one or more monosaccharide, disaccharide, oligosaccharide and/or polysaccharide. For example, said one or more saccharide may be a disaccharide. In one embodiment, said one or more saccharide may be a polysaccharide.

When the one or more saccharide is/are (a) monosaccharide, the monosaccharide can be, for example, fructose, glucose and/or galactose.

In case that the one or more saccharide is/are (a) disaccharide, the disaccharide can be, for example, trehalose or saccharose. It may also be that the disaccharide is/are maltose and/or lactose.

When the one or more saccharide is (a) polysaccharide, the polysaccharide may be, for example, dextran. In case that the one or more saccharide is an oligosaccharide, the oligosaccharide may be, for example, cyclodextrin.

For said product or method according to the present invention, said liquid composition may further comprise one or more components selected from the group consisting of excipients, additives, diluents, carbohydrate, chelating agents, surfactants, polyols, bulking agents, stabilizers, cryoprotectants, lyoprotectant(s), solubilizers, emulsifiers, salts, adjuvants, tonicity enhancing agents, delivery vehicles, lipid nanoparticles, magnetic nano- or micro-beads, reducing agents, antifoaming agents and anti-microbial preservatives.

Such buffers may be, for example, a citrate-buffer, a histidin-buffer, a Tris-buffer, a HEPES-buffer or a MOPS-buffer.

Lyophilization may subject a protein to conditions such as freezing, temperature ramps, vacuum, and dehydration. All of which can disrupt the fragile protein structure leading to loss of activity. The term "excipients" as used in the context of the present invention may mean a component that ensures that such disruption is reduced or minimized.

Cryoprotectants as used in the context of the present invention may be included into said liquid composition to protect the proteins from damage during freezing, while lyoprotectants may be present to provide protection against the stresses that occur during drying.

Said liquid composition may comprise one or more detergent(s). Such one or more detergent(s) may be (an) anionic detergent(s), (a) cationic detergent(s), (an) amphoteric detergent(s), or (a) non-ionic detergent(s).

An example for a cationic detergent may be, e.g., cetyltrimethylammoniumbromide.

As used herein, an amphoteric detergent(s) may be, for example, taurine or betain.

A non-ionic detergent, as used herein, may be, for example, a poloxamer, an octylphenoxypolyethoxyethanol, a polyethylenglycolsorbitanmonolaurat, a polyethylenglycollaurylether or nonylphenol ethoxylate, preferably a detergent from the Tween^{™}-, Brij^{™}- or Ecosurf^{™}-series.

It may also be that said liquid composition (further) comprises one or more selected from the group consisting of amino acids; preferably glycine, proline, phenylalanine, arginine, histidine or serine; peptides, nucleotides, proteins, nucleic acids, cells and (bacterial) phages.

Said liquid composition may also comprise one or more lyoprotectant(s). Such one or more lyoprotectant(s) may be one or more disaccharide, polyether or alcohol. However, such one or more lyoprotectant(s) can also be, for example, an oligosaccharide or polysaccharide. For example, the one or more lyoprotectant(s) may be selected from the group consisting of trehalose, dextran, cyclodextrin, polyethylene glycol, polyethylene glycol ester, polyvinylpyrolidone, saccharose, maltose, mannose, raffinose, fructose, glucose, galactose, lactose, and sorbitol.

Trehalose is a disaccharide formed by a 1,1-glycosidic bond between two α-glucose units. It is found in nature as a disaccharide and also as a monomer in some polymers.

Dextran is a complex branched glucan, a polysaccharide derived from the condensation of glucose. IUPAC defines dextrans as "branched poly-α-D-glucosides of microbial origin having glycosidic bonds predominantly C-1 → C-6". Dextran chains are of varying lengths, e.g. from 3 to 2000 kDa. The polymer main chain consists of α-1,6-glycosidic linkages between glucose monomers, with branches from α-1,3-linkages. This characteristic branching distinguishes a dextran from a dextrin, which is a straight chain glucose polymer tethered by α-1,4 or α-1,6 linkages.

Cyclodextrins are a family of cyclic oligosaccharides, consisting of a macrocyclic ring of glucose subunits joined by α-1,4-glycosidic bonds. Cyclodextrins are produced from starch by enzymatic conversion. Cyclodextrins are composed of 5 or more α-D-glucopyranoside units linked 1->4, like in amylose. Typical cyclodextrins contain a number of glucose monomers ranging from six to eight units in a ring, creating a cone shape.

Polyethylene glycol (also abbreviated PEG) is a polyether compound derived from petroleum. PEG is also known as polyethylene oxide (PEO) or polyoxyethylene (POE), depending on its molecular weight. The structure of PEG is commonly expressed as H-(O-CH₂-CH₂)n-OH.

Polyethylene glycol esters are the esters of PEG. Those are manufactured by reacting a polyethylene glycol with a fatty acid. The polyethylene glycol comprises the hydrophilic part of the surfactant and the fatty acid the lipophilic part.

Polyvinylpyrolidone (abbreviated PVP) can also be called polyvidone or povidone and is a water-soluble polymer of N-vinylpyrrolidone, available in a range of molecular weights and related viscosities.

Saccharose, a disaccharide, is composed of glucose and fructose subunits. It is produced naturally in plants and is the main constituent of white sugar with the molecular formula C₁₂H₂₂O₁₁.

Maltose, also known as maltobiose or malt sugar, is the disaccharide formed from two units of glucose joined with an α(1→4)-bond. Mannose, a sugar monomer of the aldohexose series, is the C-2 epimer of glucose. Raffinose, is the trisaccharide composed of galactose, glucose, and fructose. Fructose, or fruit sugar, is a ketonic sugar, often bonded to glucose to form the disaccharide sucrose. Glucose is the well known sugar with the molecular formula C₆H₁₂O₆, galactose is an aldohexose and a C-4 epimer of glucose. Lactose, or milk sugar, is the disaccharide composed of galactose and glucose and has the molecular formula C₁₂H₂₂O₁₁.

Sorbitol, also known as glucitol, is a sugar alcohol with a sweet taste which the human body metabolizes slowly. It can be obtained by reduction of glucose, which changes the converted aldehyde group (-CHO) to a primary alcohol group (-CH₂OH). Most sorbitol is made from potato starch, but it is also found in nature, for example in apples, pears, peaches, and prunes. It is converted to fructose by sorbitol-6-phosphate 2-dehydrogenase. Sorbitol is an isomer of mannitol, another sugar alcohol. The two differ only in the orientation of the hydroxyl group on carbon 2.

Said liquid composition may further comprise one or more cryoprotectant(s). Such one or more cryoprotectant(s) may be selected from the group consisting of anti-freezing peptide(s), polyethylene glycol, polyvinylpyrolidone, dextran, glycerol, DMSO, saccharose, glucose and sugar alcohols. Such sugar alcohols may be, for example, glycerine, sorbitol or ribitol. Said cryoprotectant may be comprised in said liquid composition in an amount of at most 0.5%, preferably in a concentration in the range from about 0.2 % to about 0.5 %.

In the case that said liquid composition comprises one or more reducing agent(s), such may be, for example, DMSO or DTT.

The liquid composition, as comprised in the product according to the present invention and as used in the method according to the present invention may have a pH in the range of from about 6.5 to about 10.0, preferably in the range of from about 6.5 to about 9.0.

Said liquid composition may also comprise one or more excipient(s) as defined herein. Such one or more excipient(s) may be selected from the group consisting of trehalose, cyclodextrin, dextran, and mixtures thereof.

Further, it is also within the context of the present invention that said liquid composition comprises one or more carbohydrate. Such one or more carbohydrate may be selected from the group consisting of trehalose, saccharose, maltose, mannose, raffinose, fructose, glucose, galactose and lactose. In the presence of the one or more carbohydrate it/they may be present in said liquid composition in a concentration of from about 0.5 % to about 37.5 %, preferably in a concentration of from about 1.0 % to about 35.0 %.

Further, said liquid composition may also comprise a biological, inorganic and/or organic material as being well known to the person skilled in the art.

Said liquid composition may also comprise one or more polymer, preferably a biological compatible polymer. The term biological compatible as used herein and in the context of the present invention may mean a polymer that can be used to interact with a living organism without altering its biological functioning.

Said one or more polymer(s) may has/have a molecular weight in the range from about 200 to about 35000, preferably a molecular weight in the range from about 400 to about 20000, more preferably from about 2000 to about 6000.

For example, said one or more polymer may be present in a concentration from about 0.1 % to about 40 %, preferably in a concentration from about 0.1 % to about 20 %, more preferably in a concentration from about 1 % to about 18 %, in said liquid composition.

The one or more polymer may be selected from the group consisting of polyvinylpyrrolidone (PVP), dextran and polyethylene glycol (PEG).

The general description of the invention provided above and possible embodiments thereof, as well as its purposes and features, will become more apparent with reference to the following figures and detailed description, which are directed towards exemplary embodiments of the present invention.
Fig. 1 shows a flow diagram illustrating an exemplary method of the present invention for the manufacture of frozen pellets.
Fig. 2 illustrates an exemplary device for executing the method of the present invention.
Fig. 3 shows a diagram illustrating assay performance of beads manufactured by the method of the present invention. Shown are the same qPCR beads, but tested once with a low and once with a high target concentration.

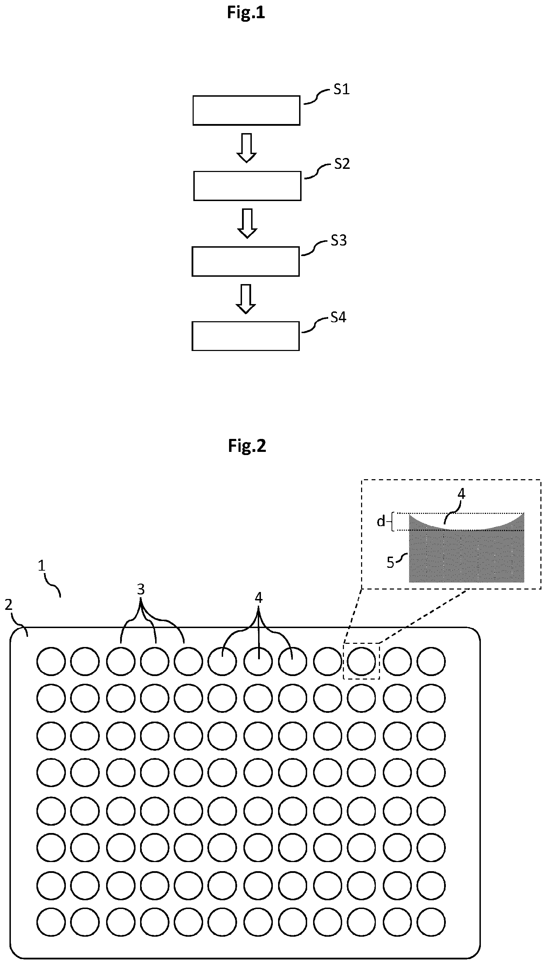

Fig. 1 shows a flow diagram illustrating an exemplary method of the present invention. In a first step S1 the method comprises applying a predefined quantity of a liquid composition to a respective receiving surface of a plurality of receiving structures. The receiving surfaces of the receiving structures have a concave shape and said liquid composition comprises one or more saccharide. In order to enable freezing of the liquid composition, the receiving surfaces are cold, i. e. they have been cooled to a predetermined temperature. In a next step S2 the method comprises freezing the liquid composition, thereby generating a plurality of frozen pellets. At this point the basic embodiment of the method terminates.

The embodiment of the method of the present invention illustrated in Fig. 1 comprises further steps which aim to manufacture freeze-dried pellets. Thus, in a next step S3 the method comprises removing frozen pellets from the receiving surfaces, for example by a removing tool which lifts or pushes the frozen pellets from the receiving surfaces as it is moved over the plurality of receiving structures. In a next step S4 the method comprises drying the frozen pellets preferably by means of lyophilisation. For this purpose, the frozen pellets may be arranged in a container in a (multi-)layered manner.

**Fig. 2** illustrates a top view of an exemplary device 1 for executing the method of the present invention. The device 1 comprises a carrier 2, such as a plate, comprising a plurality of receiving structures 3. In the illustrated example, the carrier comprises a regular arrangement of twelve rows of eight receiving structures, amounting to 96 receiving structures 3. The general size of the carrier 1 and the arrangement of the receiving structures thereon may be configured in accordance with 96-well SBS standard.

Each receiving structure 3 comprises a concave receiving surface 4 which forms the upper surface of the receiving structure 3. Each receiving surface 4 is provided to hold a certain volume of the liquid composition to be arranged thereon. In the enlarged side view which shows a receiving structure 3 in a side view, it may be seen that it is similar to a post with a concave receiving surface 4. The receiving surface is characterized by its depth d. In further embodiments of the device, the form of the receiving surface may correspond to a spherical cap. The depth d and the form of the concave receiving surface 4 may be adjusted according to need. A receiving surface 4 having the form of a spherical cap leads to spherical frozen pellets which offer the advantages described beforehand.

Generally, coolable carriers of the kind as disclosed in the international application no. PCT/EP2024/051854 may be used to carry out the method of the present method.

### EXAMPLES

### Example 1

### Production of mock beads demonstrating size and volume homogeneity of beads

For demonstrating the production capability of homogeneous mock beads with a minimum of components (e.g. only trehalose, other excipients like sucrose, PEG etc., are also possible), a volume of 10 µL was produced in the following way:
Dilution of 40 % stock solution (trehalose) with water to 10 % was performed before cryo-bead production. For cryo-bead production, the plate with an array of receiving structures of r = 1.35 mm and a = 1.0 mm, h = 0.75 mm (see Figure 1) was cooled down to -50 °C. 10 µL of 10% trehalose solution was aliquoted onto a concave receiving structure using a high precision 384-well pipetting tool with 10 µL/sec.

All 384 cryo beads were formed parallel in time within 2 sec after pipetting. After reaching -50 °C within 10 sec, the beads were released from the structured cooling plate using a bead releasing tool. Released cryo-beads were pooled as required before lyophilization. All beads produced according to the present invention show mass homogeneity of CV < 2 %.

### Example 2

### Production of qPCR beads with high assay precision

A complex qPCR Mastermix containing typical amounts of buffer, salts, detergents, Taq DNA polymerase, dNTPs, primer + probes (e.g. SARS-CoV-2 primers and probes) and 2 % of trehalose and 8 % dextran were processed as described in Example 1.

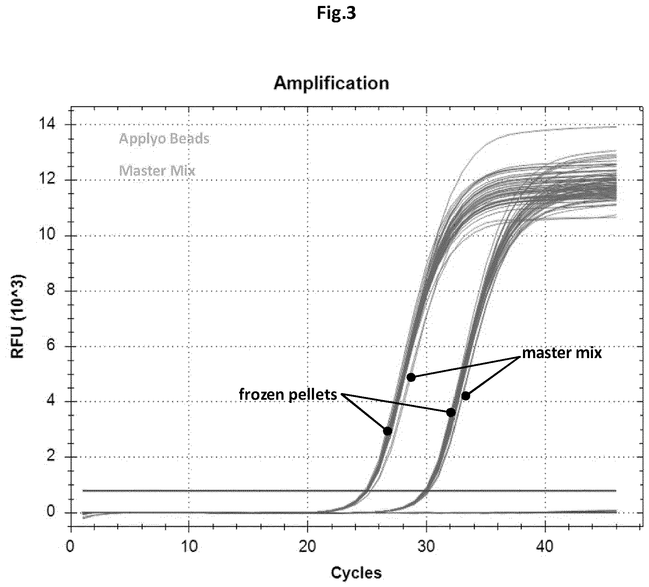

**Fig. 3** shows assay performance of SARS-CoV-2 qPCR using two concentrations of artificial SARS-CoV-2 plasmid DNA (not RNA) for liquid Master-Mix and after cryo-bead production and lyophilization. Each curve represents one individual qPCR bead which was tested with a low or with a high target concentration. The low variation of cq-value (detection point for quantification) is very tight for all individual beads. Curves also demonstrate no or negligible difference between liquid master-mix vs. lyophilized beads.

## Claims

1. Method for the manufacture of frozen pellets, comprising:
(a) applying a predefined quantity of a liquid composition to a respective receiving surface of a plurality of receiving structures,
(b) freezing the liquid composition, thereby generating a plurality of frozen pellets,
wherein the receiving surface of the receiving structure has a concave shape; and
wherein said liquid composition comprises one or more saccharide.

2. Method of claim 1,
wherein in step (b) the plurality of frozen pellets is generated in parallel, and/or
wherein in step (b) the plurality of frozen pellets is generated in a synchronized manner,
and/or wherein the receiving surface has a shape corresponding to a spherical cap.

3. Method of claim 1 or 2,
wherein in step (b) the receiving surfaces of the plurality of receiving structures are at a predefined temperature which is in the range of +/-80 K, preferably in the range of +/-50 K, more preferably in the range of +/-30 K, and more preferably in the range of +/-10 K of the freezing temperature, the eutectic temperature or glass transition temperature of the liquid composition, preferably further comprising:
increasing or lowering the temperature of the receiving surfaces from the predefined temperature for a predetermined period of time.

4. Method of any one of claims 1 to 3,
wherein the receiving surfaces define a volume which corresponds to or is less than 50% of the volumes of the liquid composition provided on the receiving surfaces, and/or
wherein step (a) comprises applying a first liquid composition and at least a second liquid composition, simultaneously or successively, to the receiving surfaces.

5. Method of any one of claims 1 to 4, further comprising: removing frozen pellets from the receiving surfaces, preferably further comprising: arranging the removed frozen pellets in a container.

6. Method of any one of claims 1 to 5, further comprising: drying the frozen pellets, preferably by means of lyophilisation, preferably wherein the shape and/or volume of the frozen pellets and the shape and/or volume of the dried frozen pellets are substantially the same.

7. Method of claim 6, wherein the frozen pellets and the dried frozen pellets have a spherical shape, preferably wherein the frozen and the dried frozen pellets have a sphericity of at least 0.97.

8. Method of any one of claims 1 to 7, wherein the frozen pellets have a first visible mark dividing its surface into two spherical caps, the first visible mark preferably comprising a line, and/or
wherein at least one, preferably all receiving surfaces comprise an emboss which is configured to leave a corresponding second visible mark on the surface of the respective frozen pellet, and/or
wherein the plurality of frozen pellets comprises at least 8, more preferably at least 12, more preferably at least 16, more preferably at least 24, more preferably at least 96, more preferably at least 384, more preferably at least 768 frozen pellets, and/or, as long as dependent on claim 6,
wherein the plurality of frozen and subsequently dried pellets has a homogenous porosity, and/or
wherein the liquid composition comprises a biological material and/or inorganic material and/or organic material.

9. Product, in the form of a plurality of frozen pellets, obtainable by the method of any one of the preceding claims.

10. Product of claim 9, wherein the plurality of frozen pellets has the same porosity, and/or comprising at least 96 frozen pellets, preferably 384 frozen pellets, and more preferably 768 frozen pellets, all comprising the same spherical shape and the same porosity, and/or wherein the frozen pellets comprise frozen and subsequently dried, preferably freeze-dried pellets, and/or wherein the frozen pellets are of the same size and shape.

11. Product of claim 9 or 10 or method of any one of claims 1 to 8, wherein said liquid composition comprises one or more selected from the group consisting of excipients, additives, diluents, buffers; preferably citrate-buffer, histidin-buffer, Tris-buffer, HEPES-buffer or MOPS-buffer; carbohydrate, chelating agents, surfactants, polyols, bulking agents, stabilizers, cryoprotectants, lyoprotectant(s), solubilizers, emulsifiers, salts, adjuvants, tonicity enhancing agents, delivery vehicles, lipid nanoparticles, magnetic micro-beads, reducing agents, antifoaming agents and anti-microbial preservatives.

12. Product of any one of claims 9 to 10 or method of any one of claims 1 to 8 or 11, wherein said liquid composition comprises one or more detergents, preferably wherein the one or more detergent(s) is/are (an) anionic detergent(s), (a) cationic detergent(s); more preferably cetyltrimethylammoniumbromide; (an) amphoteric detergent(s); more preferably taurine or betain; or (a) non-ionic detergent(s); more preferably poloxamer, octylphenoxypolyethoxyethanol, polyethylenglycolsorbitanmonolaurat, polyethylenglycollaurylether or nonylphenol ethoxylate; and/or
wherein said liquid composition (further) comprises one or more selected from the group consisting of amino acids; preferably glycine, proline, phenylalanine, arginine, histidine or serine; peptides, nucleotides, proteins, nucleic acids, cells and (bacterial) phages;
and/or
wherein said liquid composition comprises one or more lyoprotectant(s), preferably wherein the one or more lyoprotectant(s) is/are one or more disaccharide, polyether or alcohol, more preferably wherein the one or more lyoprotectant(s) is/are selected from the group consisting of trehalose, dextran, cyclodextrin, polyethylene glycol, polyethylene glycol ester, polyvinylpyrolidone, saccharose, maltose, mannose, raffinose, fructose, glucose, galactose, lactose, and sorbitol;
and/or
wherein said liquid composition comprises one or more cryoprotectant(s), preferably wherein the one or more cryoprotectant(s) is/are selected from the group consisting of anti-freezing peptide(s), polyethylene glycol, polyvinylpyrolidone, dextran, glycerol, DMSO, saccharose, glucose and sugar alcohols, more preferably glycerine, sorbitol or ribitol;
and/or
wherein said liquid composition comprises one or more reducing agent(s), preferably wherein the one or more reducing agent is DMSO or DTT.

13. Product of any one of claims 9 to 12 or method of any one of claims 1 to 8 or 11 to 12, wherein said liquid composition has a pH in the range of from about 6.5 to 10.0, preferably 6.5 to 9.0;
and/or
wherein said liquid composition comprises one or more excipient(s), preferably wherein the one or more excipient(s) is/are selected from the group consisting of trehalose, cyclodextrin, dextran, and mixtures thereof.

14. Product of any one of claims 9 to 13 or method of any one of claims 1 to 8 or 11 to 13, wherein said liquid composition comprises one or more carbohydrate, preferably wherein the one or more carbohydrate is/are selected from the group consisting of trehalose, saccharose, maltose, mannose, raffinose, fructose, glucose, galactose and lactose;
and/or
wherein the one or more carbohydrate is/are in a concentration of from about 0.5 % to about 37.5 % in said liquid composition;
and/or
wherein said liquid composition comprises a biological, inorganic and/or organic material.

15. Product of any one of claims 9 to 14 or method of any one of claims 1 to 8 or 11 to 14, wherein said liquid composition comprises one or more polymer, preferably a biological compatible polymer; preferably wherein the one or more polymer has/have a molecular weight in the range from about 200 to about 35000, more preferably a molecular weight in the range from about 400 to about 20000, even more preferably from about 2000 to 6000, and/or
preferably wherein the one or more polymer is/are present in a concentration from about 0.1 % to about 40 %, more preferably from about 0.1 % to about 20 %, even more preferably from about 1 % to about 18 %, in said liquid composition;
and/or
preferably wherein the one or more polymer is/are selected from the group consisting of polyvinylpyrrolidone (PVP), dextran and polyethylene glycol (PEG).
